# EUROPEAN PATENT APPLICATION

(11) **EP 4 563 076 A1**
(43) Date of publication of application: **04.06.2025**
(21) Application number: 23212779.5
(22) Date of filing: 28.11.2023
(51) Int. Cl.: A61B 5/00, A61B 5/021, A61B 5/0245, A61B 5/024, A61B 5/25, A61B 5/349, A61B 5/352

(54) **A WEARABLE PATCH FOR COLLECTING HEALTH-RELATED DATA, AND A METHOD OF COLLECTING SYNCHRONIZED DATA WITH SAID PATCH**

(71) Applicant: AIKON Technologies B.V., 5656 AE Eindhoven (NL)
(72) Inventor: KANAGASABAPATHI, Thirukumaran Thangaraj, 5656 AE Eindhoven (NL); VAN DEN BRAND, Jeroen, 5656 AE Eindhoven (NL); WEBER, Jan, 5656 AE Eindhoven (NL); PHOELICH, Pieter Franciscus, 5656 AE Eindhoven (NL); HIDALGO MONTANEZ, Brenda Sofia, 5656 AE Eindhoven (NL); GNANA, Siam, 5656 AE Eindhoven (NL)
(74) Representative: V.O.

(57) **Abstract**

The invention relates to a wearable patch for collecting health-related data, comprising: an electrocardiogram, ECG, sensor; a photoplethysmogram, PPG, sensor; and wherein electrodes of the ECG sensor are arranged on a bottom side of the patch that is attachable to a skin of a user, and wherein an optical window of the PPG sensor is arranged on an opposite side of the patch.

## Description

### FIELD OF THE INVENTION

The invention relates to a wearable patch for collecting health-related data. Furthermore, the invention relates to a system comprising a wearable patch for collecting said data. Moreover, the invention relates to a method of fabricating/manufacturing a wearable patch. Also, the invention relates to a method for collecting health-related data using a wearable path.

### BACKGROUND TO THE INVENTION

There is a strong need for accurate, efficient, and convenient monitoring of vital physiological parameters. Among commonly monitored parameters are heart rate and blood oxygen saturation, traditionally gauged using electrocardiogram (ECG) and photoplethysmogram (PPG) sensors, respectively. Despite the widespread adoption and utility of these sensors, the current state of the art presents several challenges that can potentially compromise the quality and reliability of data collection.

Wearable health patches used for vital sign measurements on the chest usually involves sensors for measuring electrophysiological signals such as the ECG, skin temperature, accelerometer based respiratory rate and posture estimation. Optical measurement of photoplethysmography (PPG) are not successful mainly due to the limitations in the device placement with respect to proximal blood vessels. PPG base heart rate and respiratory rates are measured mostly on the peripheral part of the body such as the arm or the finger tips. This requires additional sensing modules for data collection. Finger clip sensors, although can provide accurate skin oxygenation / SPO2 information, they are not convenient for long-term measurements. Hence, this may result in non-compliance in measurements among the patient population.

Furthermore, there are inherent complexities in synchronization, given that these devices typically operate independently. Aligning data streams from both sources can be a cumbersome task. This misalignment can, in certain situations, lead to time lags or discrepancies in readings, potentially yielding unreliable or even conflicting results.

In instances where the data from these separate devices needs to be consolidated for holistic analysis, the utilization of complex synchronization algorithms becomes imperative. Not only can these algorithms introduce additional layers of computational overhead, but they also bring forth potential sources of error. Any inaccuracies in synchronization can result in misinterpretations or misguided conclusions drawn from the consolidated data, which could be detrimental in critical health monitoring applications.

For individuals requiring regular monitoring, having to manage, maintain, and wear multiple devices can be inconvenient and even daunting. The compounded likelihood of device malfunctions, battery drainage, or simple user errors further exacerbates this challenge.

The present state of the art does not effectively address potential motion artifacts or environmental disruptions that can introduce noise into the readings. Each device may handle such disruptions differently, resulting in a compounded risk of data inconsistency when measurements from multiple devices are considered together.

### SUMMARY OF THE INVENTION

It is an object of the invention to provide for a method and a system that obviates at least one of the above mentioned drawbacks.

Additionally or alternatively, it is an object of the invention to provide for a wearable path with improved health-related data collection capabilities.

Additionally or alternatively, it is an object of the invention to provide for a wearable patch for collecting health-related data with improved accuracy and/or reliability.

Thereto, the invention provides for a wearable patch for collecting health-related data, comprising: an electrocardiogram, ECG, sensor; a photoplethysmogram, PPG, sensor; and wherein electrodes of the ECG sensor are arranged on a bottom side of the patch that is attachable to a skin of a user, and wherein an optical window of the PPG sensor is arranged on an opposite side of the patch.

The wearable patch can be used for gathering of health-related data of a user, and encompasses integral components such as an electrocardiogram (ECG) sensor and a photoplethysmogram (PPG) sensor. In its configuration, the electrodes associated with the ECG sensor are systematically positioned on the underside or bottom side of the patch. This facilitates direct contact with the user's skin once attached. Conversely, on the opposing side of this patch, an optical window corresponding to the PPG sensor has been arranged.

The wearable patch integrates both an electrocardiogram (ECG) sensor and a photoplethysmogram (PPG) sensor. The electrodes of the ECG sensor are positioned on the bottom side of the patch, ensuring direct contact with the user's skin upon attachment. Conversely, the optical window of the PPG sensor is situated on the patch's opposite side. This arrangement leads to a consolidated and compact design, facilitating simultaneous and efficient data collection from both sensors. The metrics can be captured within a singular, unified framework, avoiding the need for multiple devices and thus minimizing potential data discrepancies.

Obtaining accurate and correlated readings from different physiological metrics can be important for various applications. The disclosed wearable patch integrates at least two important health sensors - the electrocardiogram (ECG) and the photoplethysmogram (PPG). The ECG focuses on electrical activity of the heart, providing insights into cardiac health and rhythms, while the PPG offers data related to blood volume changes in microvascular beds of tissue, which can be used for determining blood oxygenation levels, among other metrics.

An advantageous unified framework is provided. A single device is used instead of two separate devices for obtaining these two sets of data.

Using separate devices could introduce challenges, such as synchronization difficulties, data consolidation challenges. Ensuring both devices are capturing data in a synchronized manner can be complex. Even minor offsets in data capture timings can result in discrepancies that could compromise the integrity of the data and any subsequent analysis. Furthermore, when data streams from two sources are to be analyzed together, merging and aligning the datasets can be challenging and time-consuming. There's also a risk of misalignment or errors during this consolidation.

Additionally, using separate devices may lead to increased user inconvenience. Wearing multiple devices can be cumbersome for the user, potentially leading to reduced compliance in continuous monitoring. This can impact the completeness and consistency of data collection.

By encapsulating both the ECG and PPG sensors within a singular device, the wearable patch provides for an advantageous solution to the above-mentioned challenges. Capturing at least these two vital health metrics within a singular framework ensures that the data is inherently synchronized, since there's a direct and consistent correlation between ECG and PPG measurements. This reduces the potential for data discrepancies and eliminates the complexities of aligning separate data streams.

Furthermore, from a user perspective, wearing a singular, integrated device enhances convenience, improving adherence to continuous monitoring protocols. This ensures more consistent and complete data capture over extended periods.

Therefore, the integrated design of the wearable patch not only simplifies the data collection process but also augments the accuracy and reliability of the captured data. The need for multiple devices is eliminated, effectively ensuring a more streamlined, efficient, and user-friendly approach to health-data collection and/or monitoring.

In some examples, the ECG sensor can comprise multiple electrodes or be made of different conductive materials. The electrodes of the ECG sensor are positioned on the underside of the patch which, when applied, can face and adhere to a user's skin. Different techniques may be used for attaching, pushing and/or holding the wearable patch against the skin. Meanwhile, on the opposing side of the patch, there exists an optical window for the PPG sensor.

Optionally, the opposite side of the patch, comprising the optical window of the PPG sensor, is configured to be on a top side of the device when worn by the user.

This orientation ensures that the optical window remains easily accessible and visible to the user. As a result, when the user wants to engage the reflection mode of the PPG sensor by placing their fingertip on it, the process becomes easier. This strategic configuration enhances the user experience, ensuring ease of access and operational efficiency.

Optionally, the wearable patch further comprises at least one additional sensor. In some examples, the additional sensor is an accelerometer. The accelerometer may be configured to detect accelerations. Additionally or alternatively, gyroscopes may be used. The wearable patch may be configured to detect movements, orientations, and/or positions. In some examples, at least a subset of the additional sensors are used for detecting falling, lying down, or absence of movement, thereby enabling alarm triggering based on detected data. Additionally or alternatively, a bioimpedance sensor as an additional sensor. The bioimpedance sensor may be configured to measure electrical impedance in the body tissues, aiding in assessing hydration, tissue health, or other physiological parameters. The data from the additional sensor is processed by the processing unit and can be stored and transmitted along with the ECG and PPG data to one or more external devices for health monitoring.

Additionally or alternatively, the wearable patch may include various other additional sensors. Further examples of additional sensors are: temperature sensor, a sweat analysis sensor, a motion sensor, a galvanic skin response sensor, a humidity sensor, a blood pressure sensor, a UV exposure sensor, etc. In some examples, the one or more additional sensors are integrated with the patch and are configured to collect additional health-related and environmental data pertaining to the user.

Optionally, the PPG sensor is configured to measure SpO2 in reflection mode by analyzing light reflected from a fingertip placed over the optical window during use.

The PPG sensor can be adeptly designed to determine the oxygen saturation level (SpO2) in reflection mode. This can be achieved by the critical analysis of light which is reflected back when a user's fingertip is purposefully placed over the sensor's optical window during operational use. This configuration provides a non-invasive means of gauging SpO2 levels, leading to quick and efficient readings while maintaining user comfort.

SpO2, or oxygen saturation levels, can be measured using transmission-based methods where light is transmitted through a body part, such as a fingertip or earlobe, and a detector on the opposite side capturing the light. However, this method can be limiting, especially for wearable devices designed for placement on larger body parts like the torso. To overcome these challenges, the PPG sensor in the wearable patch is designed to measure SpO2 using reflection mode. In this method, both the light source and detector are on the same side of the body part being measured. When a user places their fingertip over the optical window of the patch, light from the PPG sensor is emitted onto the fingertip. This light penetrates the skin and underlying tissues, and depending on the oxygen saturation in the blood, a certain amount of this light is absorbed while the rest is reflected. The sensor then detects this reflected light, and by analyzing the characteristics of the reflected light, the device can determine the oxygen saturation level, or SpO2, of the blood.

The reflection mode allows for SpO2 measurements without having to penetrate deeply into the body or requiring any invasive procedures. The fingertip simply needs to be placed over the optical window, minimizing discomfort or disruption to the user. Furthermore, given that the light source and detector are in close proximity, the time required to capture and analyze the reflected light is reduced. This ensures that the device can provide rapid feedback to the user, important for those who require real-time health monitoring. Moreover, the arrangement facilitates easy placement of a fingertip over the optical window, without the need for additional attachments or adjustments. This enhances the overall user experience, making it more that individuals will use the device consistently and correctly.

Optionally, the patch is configured to initiate a measurement on-demand by a placement of a finger on the optical window of the PPG sensor.

The wearable patch is structured to commence a measurement activity upon user initiation. This can be effectuated by the mere placement of a fingertip on the positioned optical window belonging to the PPG sensor.

The wearable patch can be arranged to initiate measurements on-demand by merely placing a fingertip on the optical window associated with the PPG sensor. This reactive design eliminates the need for complex activation procedures, ensuring a seamless and intuitive user experience.

The patch is not just a passive device but a dynamic instrument that actively responds to user gestures. Specifically, it has been arranged to recognize and initiate measurements when a user places their fingertip on the optical window linked to the PPG sensor. Advantageously, the often cumbersome and time-consuming need to navigate through multiple device settings or push buttons to start a measurement can be circumvented.

The immediacy of this on-demand activation minimizes the window of potential errors or delays, ensuring that users capture data precisely when they intend to.

In some examples, the initiation may be triggered by other external stimuli like pressing a button, applying pressure, or via a command from a connected device. Other mechanism are also possible, such as for instance a voice command.

Optionally, the patch further comprises a detection sensor configured to detect the presence of a finger, thereby activating the PPG measurement.

The detection sensor can be configured to discern the presence of a finger, a function which subsequently activates the PPG measurement process. This can further streamline the measurement initiation process. Once the finger is detected, the PPG measurement is automatically activated, negating manual activation and ensuring that the patch remains highly user-centric and responsive.

Upon detecting the presence of a finger, the PPG measurement can be initiated/activated.

Optionally, the detection sensor is a capacitive sensor. For instance, the detection sensor can be a capacitive sensor configured to sense the presence of a finger by detecting changes in capacitance upon contact with the optical window of the PPG sensor.

Capacitive sensors can provide for high sensitivity and precision in detecting subtle changes in the capacitance, especially when a conductive object, like a human finger, comes into proximity. By employing such a sensor, the device guarantees swift and accurate detection of the user's fingertip, leading to instantaneous activation of the PPG measurements.

Optionally, the detection sensor is configured to scan for the presence of a finger. In some examples, the detection sensor is configured to continuously scan for the presence of a finger over the optical window of the PPG sensor and initiate a PPG measurement upon detecting said presence. Various detection techniques may be employed.

Optionally, the detection sensor comprises a light emitting unit configured to emit light, and wherein the detection sensor is further configured to detect a reflecting signal from the emitted light, said reflecting signal being indicative of the presence of a finger over the optical window of the PPG sensor.

The detection sensor may be configured to emit light, and the sensor is adeptly designed to detect a reflective signal from this emitted light. Such a reflecting signal becomes particularly pronounced when a finger is placed over the optical window of the PPG sensor. By harnessing this reflection-based detection approach, the device can gain an added layer of accuracy in fingertip detection, ensuring that the initiation of PPG measurements remains both prompt and precise.

Other mechanisms to detect finger placement are also possible, such as for instance physical touch sensor, infrared, or even thermal sensors.

Optionally, once the finger is placed on the device, the patch is configured to enable a predetermined wavelength of LED light in the PPG module based on a detected value indicative of a skin type.

The wearable patch, upon detecting a finger placement, can engage a unique mechanism that activates a predetermined wavelength of LED light within its PPG module, a decision made contingent upon a detected value that is indicative of the user's skin type.

Recognizing the diversity of user skin types, the patch exhibits the capability to adjust its measurement parameters. Once a finger is detected and placed on the device, the patch can ascertain a skin type indicative value and subsequently modulate the wavelength of the LED light in the PPG module to suit. This adaptability ensures optimal measurement accuracy across varying user profiles.

The wearable patch embodies an improved approach to user diversity by acknowledging the variations in human skin types, which can range in color, thickness, and other physiological properties. These variations in skin types can influence the optical properties of light absorption and reflection, especially when performing photoplethysmogram (PPG) measurements that rely on light penetration through the skin.

In order to ensure that the PPG measurements are not biased or compromised by these skin type variations, the patch is designed with a dynamic adaptability feature. Upon the placement of a finger on the device, the patch's integrated sensors can determine an indicative value that corresponds to the user's specific skin type. This indicative value can be derived from various factors, such as the initial light absorption and reflection properties of the skin under test.

Once this skin type indicative value is determined, the patch can modify or "modulate" the wavelength of the LED light used in the PPG module. For instance, darker skin types may require a different LED wavelength compared to lighter skin types to achieve optimal light penetration and reflection. By adjusting the LED wavelength, the device can effectively ensure that the PPG measurements are tailored to the individual user, thereby minimizing potential inaccuracies.

The ability to adjust the LED wavelength in real-time, based on the detected skin type, represents a significant advantage. This dynamic adaptability ensures that the device can be effectively used for a broad spectrum of users, ensuring consistent and optimal measurement accuracy irrespective of skin type variations. Such precision in measurements is critical in health monitoring, as it provides more reliable data.

In some examples, the device can leverage multiple LED wavelengths, adjust the light intensity, and/or use environmental factors to optimize the reading for varying skin properties, tones and/or conditions.

Optionally, the patch comprises a common device clock used for synchronizing measurements.

Eliminating other synchronization techniques lead to significantly reduced computational needs, which in turn leads to energy savings, a critical factor for wearable medical devices.

The common device clock can serve the important purpose of ensuring that measurements across the device, from at least the ECG and PPG sensors, are accurately synchronized. By centralizing the reference time point within the same device, data consistency is maintained, eliminating potential discrepancies and enhancing overall measurement reliability.

The timing of data collection is important. Consider the ECG and PPG sensors working independently, each with its own clock. In such a setup, even slight discrepancies in timekeeping can lead to misalignment of data, causing inaccuracies in derived metrics like Pulse Arrival Time (PAT). Such misalignments, albeit small, can drastically alter a user's health profile, leading to potential misdiagnoses or incorrect health recommendations. By deploying a singular, unified common device clock, the wearable patch ensures that data collected from the ECG sensor and the PPG sensor are anchored to the same temporal reference point. This means that each data point, whether it be an ECG waveform or a PPG waveform, is timestamped in relation to this centralized clock, guaranteeing their temporal alignment.

Furthermore, by centralizing the reference time point within the device, a seamless synchronization is achieved. This does away with the need for complex external synchronization algorithms or processes that may be required if data were sourced from separate devices or systems. Such processes not only introduce computational overhead but also risk introducing errors.

The integration of a common device clock in the wearable patch offers an advantage by ensuring consistent and synchronous data collection from both sensors of the wearable patch. This centralization avoids potential time-based discrepancies, thereby significantly enhancing the reliability of the measurements.

The wearable patch can encompass a shared device clock. In some alternative examples, an external time synchronization unit is used that is configured to perform time synchronization through a wireless connection.

Optionally, the wearable patch is configured to synchronize measured signals of ECG and PPG sensors.

Optionally, the patch comprises a synchronization module configured to concurrently align and coordinate the data streams from the ECG and PPG sensors. In this way, real-time coherency between the measured signals of both sensors can be ensured.

The wearable patch may be configured to employ the synchronization module to align and coordinate the data streams from both the ECG and PPG sensors concurrently. This simultaneous coordination ensures that there's real-time coherency between the signals derived from both sensors. By leveraging this module, potential delays or misalignments between the two data streams are effectively mitigated. The result is an enhanced accuracy in health metric derivation, ensuring that the signals from both sensors are harmoniously integrated, thus providing reliable and cohesive health assessments.

Optionally, the patch is configured to employ the common device clock for timestamping collected data.

The wearable patch may utilize the in-built common device clock not only for synchronization but also for the task of timestamping the gathered data, ensuring each data point is chronologically logged.

An additional layer of data accuracy can be obtained in this way, ensuring that each data point is correctly associated with its respective collection time, thus facilitating precise health monitoring. Timestamping is the process of associating a specific time or date with a piece of data at the moment of its collection. In the context of the wearable patch, as it collects health-related data such as ECG and PPG readings, the common device clock ensures that every single data point captured is paired with the exact time of its collection.

By ensuring every piece of data is associated with its exact collection time, the patch ensures the temporal accuracy of the readings. This means that health metrics can be traced back to specific moments in time, which can be important in monitoring certain conditions or events. Furthermore, this timestamping function enhances the reliability of the data. In medical monitoring, especially where continuous data streams are involved, knowing the sequence and exact time of events is important. For instance, understanding the precise temporal relationship between an abnormal ECG reading and a subsequent PPG reading can provide important insights into a patient's cardiovascular health.

By providing a consistent temporal reference across all sensors, the patch ensures the synchronized collection of data, thereby negating any chances of misinterpretation due to temporal misalignment.

Optionally, data collected using the ECG sensor and data collected using the PPG sensor is timestamped and correlated to measure Pulse Arrival Time (PAT).

The data that the ECG sensor accumulates, when juxtaposed with that from the PPG sensor, undergoes a process where both are timestamped and subsequently correlated. This enables the precise determination of the PAT.

By sourcing both sets of data from the same device, potential synchronization inconsistencies are mitigated, leading to reliable PAT measurements. The wearable patch offers the capacity to evaluate advanced health parameters, a prime example being the PAT, which is a significant metric, representing the time taken for the pulse wave to travel between two arterial sites, commonly used in cardiovascular health assessments and blood pressure estimation.

The ECG sensor captures the electrical activity of the heart, marking the initiation of the pulse wave. Meanwhile, the PPG sensor detects the arrival of this pulse wave by observing changes in blood volume within microvascular tissues. For accurate PAT calculation, it is important that these two data points are perfectly synchronized in time. By virtue of the patch's design, data from both these sensors is timestamped using a common device clock, ensuring a synchronized reference for each data point. This uniform timestamping is important in correlating the initiation (from ECG) and arrival (from PPG) of the pulse wave, forming the foundation for precise PAT computation.

Furthermore, both ECG and PPG sensors can be housed within a singular device. Synchronization challenges often arising due to minor time lags or discrepancies between the clocks of the separate devices, that can adversely impact the accuracy of PAT measurements, can be effectively avoided since both sets of data originate from the same device utilizing a shared clock.

In some examples, different algorithms or mathematical models may be employed to calculate PAT, ensuring adaptability to diverse health conditions and user profiles.

Optionally, the ECG sensor is configured to detect electrical activity of the heart and produce an ECG waveform, wherein the PPG sensor is configured to optically measure blood volume changes in the microvascular bed of tissue and produce a PPG waveform, and wherein the common device clock is operably connected to both said ECG sensor and said PPG sensor, and wherein the common device clock is configured to provide synchronized timestamping for both the ECG waveform and the PPG waveform, and wherein a processing unit of the patch is configured to calculate the PAT based on the synchronized timestamped data from both the ECG and PPG waveforms.

The ECG sensor can be precisely arranged to capture the heart's electrical activity, producing an ECG waveform in the process. Concurrently, the PPG sensor, through optical means, can quantify blood volume alterations in the microvascular bed of the tissue, thereby generating a PPG waveform. A common device clock, being functionally interconnected with both the ECG and PPG sensors, provides synchronized timestamps for the ECG and PPG waveforms. A dedicated processing unit in the patch utilizes this synchronized data, subsequently calculating the PAT.

The shared common device clock can connect to both sensors, facilitating synchronized timestamping for both waveforms. As a result, a dedicated processing unit in the patch can calculate PAT based on this synchronized data, ensuring measurement cohesion and accuracy.

With the data being accurately timestamped and synchronized, the patch's dedicated processing unit can be tasked with the computation of the PAT. By comparing specific points on the synchronized ECG and PPG waveforms, the processing unit calculates the PAT.

A processing unit within the patch is tasked with computing the PAT based on the synchronized data from these waveforms. In some examples, the processing unit can be an embedded microcontroller, a FPGA, or even a cloud-based processor, each offering varied levels of computational capabilities.

Optionally, the processing unit is further configured to derive PAT as the time difference between a point on the ECG waveform, and a point on the PPG waveform.

The PAT is computed by determining the temporal difference between a particular point on the electrocardiogram (ECG) waveform and a corresponding point on the photoplethysmogram (PPG) waveform. The ECG waveform, capturing the heart's electrical activity, serves as an initial reference point, marking the heart's contraction and subsequent initiation of a blood pulse. On the other hand, the PPG waveform, resulting from optically measuring blood volume changes in the microvascular bed of tissue, signifies the time the pulse reaches peripheral capillaries.

By measuring the time interval between these two specific points across the two waveforms, the processing unit calculates the PAT. This interval is indicative of the time taken for a pulse wave to travel from the heart to the peripheral vasculature, providing invaluable insights into cardiovascular health and arterial stiffness. By harnessing data from both the ECG and PPG sensors integrated within the same device, potential synchronization errors or discrepancies are markedly reduced, ensuring the derived PAT's reliability and accuracy.

The processing unit, in its function, derives the PAT as the time differential between a specific point on the ECG waveform and a corresponding point on the PPG waveform. In alternative examples, the chosen points may vary, or multiple points may be used to derive an average PAT value.

Optionally, further comprising a storage module configured to store data, and wherein a wireless communication module is provided that is configured to transmit data to one or more external devices.

The combination of the storage and wireless communication modules in the wearable patch results in a dual-feature design. On one hand, there's an assurance of secure, in-device data storage, which is important for retaining comprehensive health logs over extended periods. On the other hand, the ability to transmit this stored data wirelessly ensures that, when needed, the data is effortlessly accessible, be it for immediate health assessments, long-term health trend analysis, or sharing with medical professionals. This cohesive integration guarantees that users not only benefit from continuous health tracking but also from the convenience of accessing, analyzing, and sharing their health metrics without any expert.

In some examples, storage can be flash memory, EEPROM, cloud storage, etc., while the communication module can operate via Wi-Fi, Bluetooth, NFC, cellular networks, etc.

In some examples, the wearable patch further comprises: a battery for powering components of the patch; a recharging mechanism operably connected to said battery for replenishing its energy levels; and a communication interface for transmitting collected health-related data.

For example, the communication interface may be configured to support 5G, WiFi, any other wireless communication protocol and/or wired network connectivity. In some examples, the wearable patch is configured to transmit collected health-related data to a storage (e.g. a cloud-based storage) and/or an analysis platform.

Optionally, the wearable patch includes a flexible foil that has an adhesive layer on a side for attachment to the skin of the user.

The wearable patch's design can incorporate a pliable foil, a feature that facilitates its adaptability to the body's contours. This foil possesses an adhesive layer on one side, ensuring that once applied, it remains steadfastly attached to the user's skin.

The flexible foil can be augmented with an adhesive layer on one side, ensuring that the patch remains securely attached to the user's skin. This design ensures prolonged wearability without causing user discomfort, ensuring continuous data collection.

In addressing the important aspects of user experience and comfort, the disclosed wearable patch can be designed with an emphasis on flexibility and adaptability. Constructed primarily using a flexible foil, the patch can easily conform to the contours of a user's body, ensuring that it does not restrict movement or feel rigid upon application. This flexibility is instrumental in allowing the user to go about their daily activities unencumbered, fostering a sense of wearing something natural rather than a foreign object.

In some examples, the patch is made from biocompatible silicone, elastomers, or other flexible materials, and may use hypoallergenic adhesives or mechanical fasteners for skin attachment. Various implementations are possible.

The wearable patch can be affixed to various body locations.

Optionally, the patch is configured to be worn on the torso, for example on a chest and/or a lateral left side of the body.

This positioning ensures optimal contact with the heart's electrical activity, ensuring that the ECG measurements are both accurate and consistent. The wearable patch's design takes into account both ergonomic and functional considerations. Specifically, its positioning has been determined to be on the torso, with a primary focus on the chest area or the lateral left side of the body.

Such a location is rooted in the anatomical proximity to the heart. By placing the patch close to the heart, it can more effectively and directly capture the heart's electrical signals. This strategic placement maximizes the fidelity and clarity of the electrocardiogram readings. It ensures that external interference and potential attenuation of the signal due to distance or intervening tissue layers are minimized. As a result, the ECG measurements derived from the patch when worn in this location are not only accurate, reflecting the true cardiac electrical activity, but also consistent, ensuring that repeat measurements are reliable and comparable. This positioning also augments user comfort, reducing the likelihood of inadvertent patch displacement during daily activities, and ensuring sustained quality of data collection.

The placement of the patch is thus important for reliable, high-resolution heart monitoring in real-world scenarios. In some examples, the patch may have varying sizes and/or shapes to cater to different body types and anatomical considerations.

According to an aspect, the invention provides for a method of fabricating a wearable patch for collecting health-related data, the method including: providing an electrocardiogram, ECG, sensor; providing a photoplethysmogram, PPG, sensor; and wherein electrodes of the ECG sensor are arranged on a bottom side of the patch that is attachable to a skin of a user, and wherein an optical window of the PPG sensor is arranged on an opposite side of the patch.

The ECG sensor electrodes are designed to capture the electrical impulses of the heart, making their placement on the wearable patch important for accurate data acquisition. To this end, the electrodes are placed on the side of the patch that directly interfaces with the skin when attached. This ensures that, upon application of the patch to the user's body, the electrodes establish a direct and uninterrupted contact with the skin, allowing for a consistent and reliable capture of cardiac electrical activity.

The PPG sensor operates by emitting light through an optical window, which then measures the variation in light absorption or reflection caused by blood volume changes. The optical window of the PPG sensor is aligned on the opposite side of the patch, away from the skin. This arrangement ensures that when a user interacts with the patch, typically by placing a fingertip over the optical window, there is a clear path for light to penetrate and reflect, ensuring optimal photoplethysmogram data acquisition.

By distinctly separating the ECG and PPG sensors on opposing sides of the patch during fabrication, the device ensures that there is no interference between the two sensors' operations. This spatial separation allows each sensor to operate at its optimal efficiency without any potential cross-talk or interference, leading to precise and high-quality health data collection.

Optionally, the wearable patch has one or more electrical ECG sensors on its top side. The accuracy of the ECG sensors can be further enhanced in this way. The electrical ECG sensors on the top side may be arranged at or in proximity of the optical window. Additionally or alternatively, the wearable patch may be configured to receive two fingers simultaneously, one used for contacting the optical window, the other used for contacting the electrical ECG sensors on the top side. Various different configurations are possible.

According to an aspect, the invention provides for a method for collecting health-related data using a wearable patch, the method comprising: providing a wearable patch comprising an electrocardiogram, ECG, sensor, and a photoplethysmogram, PPG, sensor, wherein electrodes of the ECG sensor are arranged on a bottom side of the patch, and wherein an optical window of the PPG sensor is arranged on an opposite side of the patch; attaching the wearable patch to a skin of a user such that electrodes of the ECG sensor on the bottom side of the patch is positioned in contact with the skin of the user; collecting electrocardiogram data via the ECG sensor; collecting photoplethysmogram data via the PPG sensor; transmitting the collected electrocardiogram data and photoplethysmogram data to a processing device for analysis and monitoring of health-related metrics.

Once the patch is securely in place, it enters its data collection mode. Both the ECG and PPG sensors operate simultaneously, collecting health metrics. The ECG sensor diligently monitors the electrical activity of the heart, translating it into ECG waveforms. In tandem, the PPG sensor uses its optical window to measure blood volume changes in the user's microvascular bed of tissue, providing insights into parameters such as blood oxygen saturation.

As data is continuously acquired, the wearable patch isn't limited to just data collection. Integrated within the patch is a wireless communication module, which plays an important role in data management and accessibility. Data collected by the patch is relayed in real-time or batches, depending on the patch's configuration, to an external processing device. This can be a dedicated medical device, a smartphone application, or a remote monitoring station. The wireless transmission ensures that the user is unhindered by cables, enhancing the mobility and overall user experience.

By facilitating the wireless transfer of this data to external processing devices, it ensures that health professionals, caregivers, or the users themselves have instant access to important health metrics, enabling timely interventions, continuous monitoring, and informed health evaluations.

In some examples, the method can incorporate real-time feedback mechanisms, alert systems, or even integrate with telemedicine platforms for comprehensive healthcare delivery.

According to an aspect, the invention provides for a system comprising a wearable patch according to the disclosure. Optionally, the system comprises a hub that is in communication, preferably wirelessly, with the wearable patch. Optionally, the wearable patch is rechargeable by means of the hub.

According to an aspect, the invention provides for and outward-facing PPG sensor integrated in a wearable health patch. The PPG sensor may be configured to perform SPO2 and/or Pulse Transit Time measurements.

It will be appreciated that any of the aspects, features and options described in view of the wearable path apply equally to the methods and the described system, devices and use. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of a cross-sectional view of an embodiment of a wearable patch;
Fig. 2 shows a schematic diagram of a cross-sectional view of an embodiment of a wearable patch;
Fig. 3 shows a schematic diagram of a top view of an embodiment of a wearable patch;
Fig. 4 shows a schematic diagram of an embodiment of a wearable patch; and
Fig. 5 shows a schematic diagram of a method.

### DETAILED DESCRIPTION

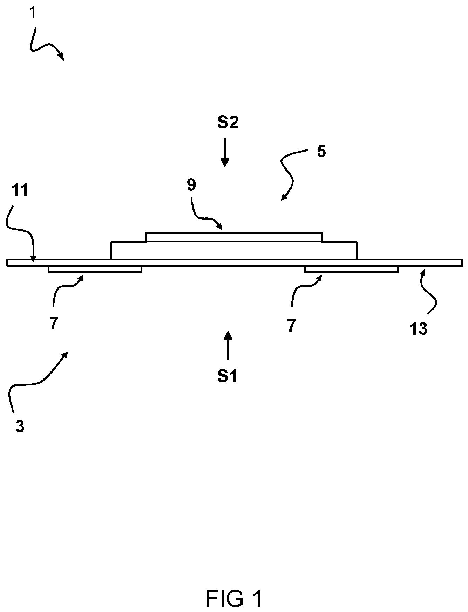

Fig. 1 shows a schematic diagram of a cross-sectional view of an embodiment of a wearable patch 1 configured to collect health-related data. The wearable patch comprises a ECG sensor 3 and a PPG sensor 5. The electrodes 7 of the ECG sensor 3 are arranged on a bottom side S1 of the patch 1 that is attachable to a skin of a user. An optical window 9 of the PPG sensor 5 is arranged on an opposite side S2 of the patch 1.

In this example, the wearable patch has a flexible foil 11 that has an adhesive layer 13 on a side for attachment to the skin of the user.

By integrating both the ECG and PPG sensors in a single device, a shared reference time point can be ensured. Since both data streams come from the same device, a direct and consistent correlation between ECG and PPG measurements can be effectively formed. There's no need for complex synchronization algorithms to align data from two devices, ensuring accuracy and reliability and reducing potential errors.

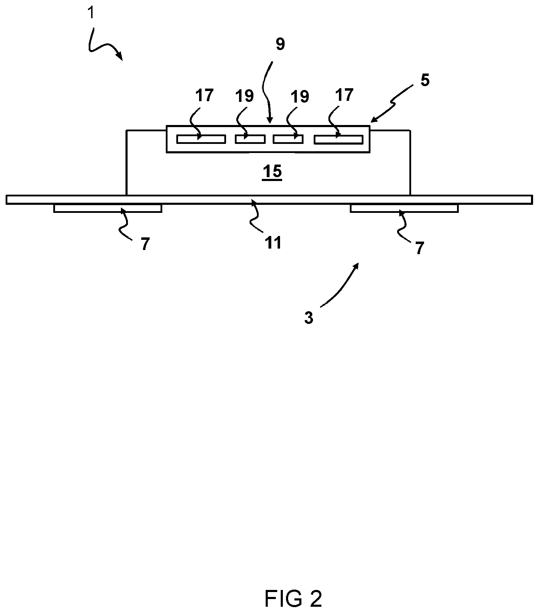

Fig. 2 shows a schematic diagram of a cross-sectional view of an embodiment of a health data collecting wearable patch 1 with an ECG sensor 3 with electrodes 7 on the skin-attachable bottom side, and a PPG sensor 5 with an optical window 9 on the opposite/top side.

In the shown example, the wearable patch 1 has an electronic unit 15. The electronic unit 15 includes readout electronic arrangements. The electronic unit 15 may comprise one or more power sources. For example, the electronic unit 15 may include a rechargeable battery unit. Additionally, the electronic unit 15 may include one or more other sensors, such as accelerometers, temperature sensors, electronic gyroscope unit, etc.

In some examples, the PPG sensor 5 has a transparent optical window 9. For example, the optical window 9 may be made of glass or other suitable materials.

In this example, the PPG sensor 5 has two light emitting units 17, such as light emitting diodes (LEDs), and two optical sensors 19, such as photodiodes.

Furthermore, in this example, the flexible foil 11 is a skin contact patch. On the bottom side thereof, skin contact electrodes 7 of the ECG sensor 3 are arranged. The wearable patch forms an integrated wearable device.

In this example, the wearable patch integrates an optical PPG module with LEDs of multiple wavelengths and one or more photodiodes on the top surface of the health patch device. To make a measurement, patients are required to place their fingers to cover the optical window on the device. Once the finger is placed on the device, depending on the skin type and/or other predetermined light-skin interaction models, a particular wavelength of LED light is enabled in the PPG module. The reflected light from the skin is measured by one or more photodiodes located on the top surface of the device.

In some examples, the wearable device 1 includes one or more other sensing elements such as bio-impedance, temperature sensor that requires electrodes to be in contact with the skin and other sensing elements such as a microphone that requires close proximity to the body. In some examples, the PPG module includes one or more LEDs of different wavelengths (Blue, Green, Red and Infra-red) and one or more photodiodes to measure the reflected light from the skin.

In some examples, the device 1 can be worn on the torso on the chest or the lateral left side of the body depending on the clinical requirements. Whenever the patient is required to make a SPO2 measurement, the patient will place a finger on top of the device. On contact, the LED gets activated, the reflected light from the finger will be measured by photodiodes in the center of the PPG module. Depending on the skin type, a particular wavelength of LED light will be used for measurement.

One or more LEDs can be used in sequence and the best signal output with low signal-to-noise ratio depending on the skin type can be used for SPO2 calculation. A capacitive touch sensor can be included in the PPG module to activate the LED lights on contact. However, other mechanisms may also be used. A particular protocol for activation such a single-tap or double-tap can be incorporated in the measurement protocol to avoid accidental light activation. The PPG signal from this integrated device can also be used for measuring the time lapsed heart rate compared to the electrodes on the bottom of the device and can thus be used for computing the pulse arrival time or pulse transit time.

In some examples, SPO2 is measured in reflection mode by measuring the light reflected from the finger-tip. The invention avoids the need to have a wired finger clip sensor to be connected to the fingers each time. SPO2 can be measured on demand by placing the fingers on top of the device. SPO2 measurement can be prompted by suitable notification provided in the patient facing mobile applications.

Advantageously, SPO2 spot measurements can be simplified. A wearable device used for continuous electrophysiological measurements can now be used for SPO2 measurement as well. The PPG module can be enabled on demand whenever a measurement needs to be performed. The data from the PPG module can be streamlined and transferred along with the rest of the wearable device data. Combining both ECG and PPG measurement in a single device with a common device clock, the data can be timestamped and correlated accurately to measure Pulse Arrival Time (PAT) in a single device. Hence, the need for a separate finger clip sensor both in clinical and non-clinical applications can be avoided.

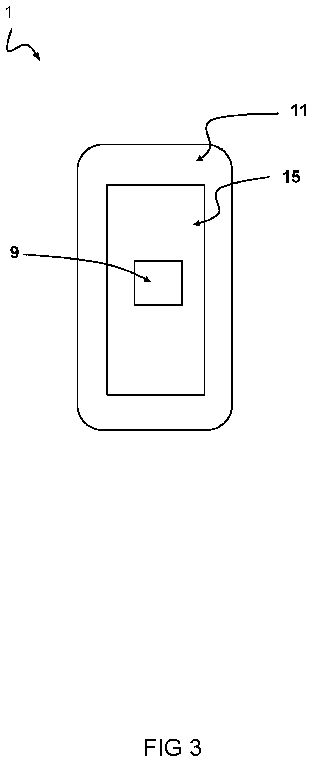

Fig. 3 shows a schematic diagram of a top view of an embodiment of a wearable patch 1. The shapes and dimensions of the wearable patch may vary. For example, the outer shape of the flexible foil 11 may be different, the shape of the optical window 9 may be different (e.g. rounded), the electronic unit 15 may be smaller relative to the optical window 9, etc. Various other configurations are envisaged.

Advantageously, the wearable patch 1 can effectively avoid inherent complexities of synchronizing data streams, and the reliance on error-prone algorithms for synchronization. Furthermore, the inconvenience of managing multiple devices, and the vulnerabilities to external disruptions can be avoided.

The wearable patch may have a common device clock.

In some examples, such that both ECG and PPG measurements have the same time resolution. In this way, both measurements may be sampled at precisely defined intervals, ensuring that the timestamps for both are congruent.

All clocks have some degree of drift over time, which is the deviation of the clock from the reference time. If ECG and PPG measurements were governed by separate clocks, even minor drifts in either clock over time would compound, causing discrepancies for example in PAT measurements.

When merging data streams from separate devices, there's often a need to re-sample, interpolate, or use synchronization algorithms to align the data. This not only introduces computational complexity but also potential sources of error. A common clock eliminates this need. Moreover, each stage of data processing can introduce errors, and these errors can compound. By eliminating the need for synchronization processes, the number of stages where errors can be introduced can be reduced.

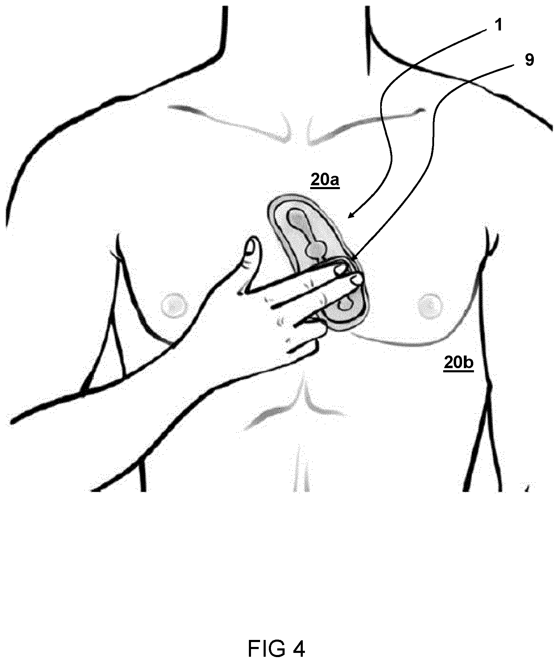

Fig. 4 shows a schematic diagram of an embodiment of a wearable patch 1. In this example, the patch is configured to be worn on the torso, for example on a chest 20a and/or a lateral left side 20b of the body. In this figure, two fingers are placed on the optical window 9 of the PPG sensor of the wearable patch 1.

The integration of the ECG and PPG sensors into a unified platform not only enhances the efficiency of data collection but also amplifies the precision and dependability of the results.

The integrated ECG and PPG sensors can have a shared reference time point. Potential synchronization challenges due to potential time lags, misalignments, or discrepancies inherent to each device's separate recording, can be effectively prevented. The ECG sensor 3 comprises electrodes positioned on the bottom side of the wearable patch 1, making direct contact with the user's skin when attached. Oppositely, the PPG sensor 5, equipped with an optical window 9, is oriented on the patch's opposing side. This configuration facilitates simultaneous data collection from both sensors while the patch is worn, ensuring a shared reference time point.

There can be a direct and consistent correlation between ECG and PPG measurements due to the co-localization of the ECG and PPG sensors within the same device. The intrinsic relationship guarantees that the data from both sensors is inherently synchronized, obviating the need for supplementary synchronization mechanisms or algorithms. In situations where external factors, such as motion artifacts or other environmental disruptions, can lead to discrepancies in data readings, this direct correlation ensures a reduced margin of error and enhances the reliability of the measurements.

Furthermore, the wearable patch inherently provides synchronized readings from both the ECG and PPG sensors. This design eliminates the necessity for, and inherent risks associated with, the deployment of additional synchronization tools. By doing so, the device ensures higher data accuracy and reliability, vital for health monitoring applications where precision is important.

The consolidated architecture with shared reference time point and streamlined approach not only enhances the efficiency of data collection but also ensures that potential errors, misalignments, or discrepancies, which can compromise the interpretation and utility of the collected data, are substantially minimized.

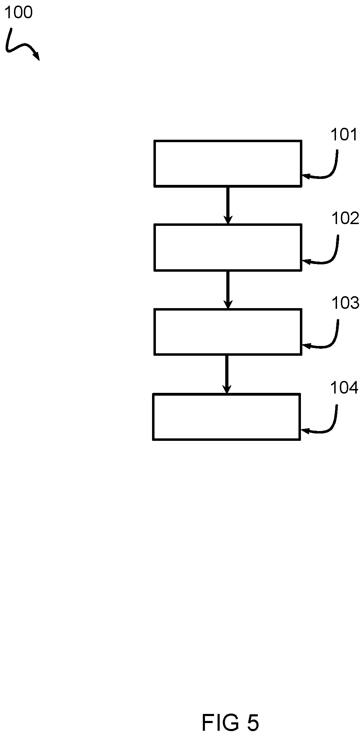

Fig. 5 shows a schematic diagram of a method 100 for collecting health-related data using a wearable patch. In a first step 101, a wearable patch comprising an ECG sensor and PPG sensor is provided, wherein electrodes of the ECG sensor are arranged on a bottom side of the patch, and wherein an optical window of the PPG sensor is arranged on an opposite side of the patch. In a second step 102, the wearable patch is attached to a skin of a user such that electrodes of the ECG sensor on the bottom side of the patch is positioned in contact with the skin of the user. In a third step 103, electrocardiogram data via the ECG sensor is collected, and photoplethysmogram data via the PPG sensor is collected. In a fourth step 104, the collected electrocardiogram data and photoplethysmogram data is transmitted to a processing device for analysis and monitoring of health-related metrics.

In some examples, various parameters such as ECG, heart rate, and breathing rate, etc. can be measured by means of the wearable patch. On the side in contact with the skin (backside), electrical electrodes may be arranged for measuring electrical signals. On the top of the device, an optical window may be arranged, which can for instance integrate photodiodes and LEDs. In some examples, measurements can be achieved with a single finger for optical readings. But, for combining electrical and optical signals, two fingers might be necessary in some examples.

The wearable patch thus has sensors on top and bottom. Advantageously, users only need to place their finger on the device for a certain period of time (e.g. 30 seconds) to get a measurement. The optical sensor on top of the device may operate in reflection mode. In transmission mode, light from LEDs reflects back to photodiodes.

The wearable patch can be made from a stretchable material, providing flexibility (e.g. flexible foil). The patch can be designed to be attached to the body. In some examples, the wearable patch may be configured to be attached to the middle section of the torso or the lateral left side of the body. This ensures a stable position with minimal motion interference. The patch may be dimensioned and/or shaped to facilitate such attachment.

The configuration and placement of LEDs and sensors on the patch can be chosen so as to ensure said LEDs and sensors fit within the finger size. The diode may be positioned in the middle, surrounded by a number of different LEDs, for example arranged around it.

Both ECG (an electrical signal representing the heart's electrical activity) and PPG (an optical signal that measures pulse rate using light) can be measured in a synchronized manner. To ensure the precision of this measurement, an internal clock may be employed to synchronize the two sensors. This synchronization ensures that any deviation in the measurement is due to the signal and not internal shifts. The wearable patch can provide two reference points for measuring the pulse's transition.

To tackle synchronization challenges due to drift, which can be understood as shifts in signal frequency or shape that can occur over microseconds, which becomes problematic when the devices are trying to capture and align signals in the milliseconds range, the wearable patch according to the disclosure provides for a unified device with a single master clock. This device would house both the ECG and PPG sensors. By using one master clock, the ECG and PPG signals can be seamlessly synchronized. This eliminates the inconsistencies that might arise from using two separate devices with their own clocks. Additionally, the device's user-friendly design would allow activation via finger touch, removing the need for auxiliary activation methods or external applications.

It will be appreciated that the wearable patch may also integrate one or more other sensors. One exemplary sensor is a bioimpedance sensor. Bioimpedance indicates changes in the impedance of the body, which fluctuates as an individual breathes. This can be a potential diagnostic tool, as changes in bioimpedance could suggest fluid accumulation in the lungs, possibly due to conditions like asthma.

Additionally or alternatively, the wearable patch may include an accelerometer for measuring motion. This can be used to determine the posture of the person. Advantageously, this helps in determining the best times to capture signals, e.g., when the person is stationary. In some examples, the accelerometers are used to identify when the person is stationary, helping in avoiding measurements during high-motion activities. Furthermore, the accelerometer's data can be used to remove motion artifacts from the ECG, based on detected movement.

Additionally or alternatively, the wearable patch may include a gyroscope. The gyroscope may be configured to determine an orientation of the person, e.g., if they are lying on their side, flat, etc.

Multiple sensors may be used for determining certain parameters. For example, the wearable patch may be configured to have one or more sensors to determine a value indicative of a respiratory rate. This can be important for assessing the general condition of the patient.

In some examples, the wearable patch is waterproof.

In some examples, the wearable patch has one or more electrical ECG sensors on its top side. For instance, the wearable patch may have five electrodes for ECG measurements: two at the top, two at the bottom, and a reference electrode in the middle. In some examples, the bottom electrodes are used to measure respiratory rates using bioimpedance techniques.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The term "and/or" includes any and all combinations of one or more of the associated listed items. The mere fact that certain measures are recited in mutually different claims does not indicate that a combination of these measures cannot be used to an advantage.

## Claims

1. A wearable patch for collecting health-related data, comprising:
an electrocardiogram, ECG, sensor;
a photoplethysmogram, PPG, sensor; and
wherein electrodes of the ECG sensor are arranged on a bottom side of the patch that is attachable to a skin of a user, and wherein an optical window of the PPG sensor is arranged on an opposite side of the patch.

2. The wearable patch of claim 1, wherein the PPG sensor is configured to measure SpO2 in reflection mode by analyzing light reflected from a fingertip placed over the optical window during use.

3. The wearable patch of claim 2, wherein the patch is configured to initiate a measurement on-demand by a placement of a finger on the optical window of the PPG sensor.

4. The wearable patch of claim 2 or 3, wherein the patch further comprises a detection sensor configured to detect the presence of a finger, thereby activating the PPG measurement.

5. The wearable patch of claim 2, 3 or 4, wherein once the finger is placed on the device, the patch is configured to enable a predetermined wavelength of LED light in the PPG module based on a detected value indicative of a skin type.

6. The wearable patch according to any one of the preceding claims, wherein the patch comprises a common device clock used for synchronizing measurements.

7. The wearable patch according to claim 6, wherein the patch is configured to employ the common device clock for timestamping collected data.

8. The wearable patch according to claim 6 or 7, wherein data collected using the ECG sensor and data collected using the PPG sensor is timestamped and correlated to measure Pulse Arrival Time (PAT).

9. The wearable patch according to claim 8, wherein the ECG sensor is configured to detect electrical activity of the heart and produce an ECG waveform, wherein the PPG sensor is configured to optically measure blood volume changes in the microvascular bed of tissue and produce a PPG waveform, and wherein the common device clock is operably connected to both said ECG sensor and said PPG sensor, and wherein the common device clock is configured to provide synchronized timestamping for both the ECG waveform and the PPG waveform, and wherein a processing unit of the patch is configured to calculate the PAT based on the synchronized timestamped data from both the ECG and PPG waveforms.

10. The wearable patch according to any one of the preceding claims, wherein the processing unit is further configured to derive PAT as the time difference between a point on the ECG waveform, and a point on the PPG waveform.

11. The wearable patch according to any one of the preceding claims, further comprising a storage module configured to store data, and wherein a wireless communication module is provided that is configured to transmit data to one or more external devices.

12. The wearable patch according to any one of the preceding claims, wherein the wearable patch includes a flexible foil that has an adhesive layer on a side for attachment to the skin of the user.

13. The wearable patch according to any one of the preceding claims, wherein the patch is configured to be worn on the torso, for example on a chest and/or a lateral left side of the body.

14. A method of fabricating a wearable patch for collecting health-related data, the method including:
providing an electrocardiogram, ECG, sensor;
providing a photoplethysmogram, PPG, sensor; and
wherein electrodes of the ECG sensor are arranged on a bottom side of the patch that is attachable to a skin of a user, and wherein an optical window of the PPG sensor is arranged on an opposite side of the patch.

15. A method for collecting health-related data using a wearable patch, the method comprising:
providing a wearable patch comprising an electrocardiogram, ECG, sensor, and
a photoplethysmogram, PPG, sensor, wherein electrodes of the ECG sensor are arranged on a bottom side of the patch, and wherein an optical window of the PPG sensor is arranged on an opposite side of the patch;
attaching the wearable patch to a skin of a user such that electrodes of the ECG sensor on the bottom side of the patch is positioned in contact with the skin of the user;
collecting electrocardiogram data via the ECG sensor;
collecting photoplethysmogram data via the PPG sensor;
transmitting the collected electrocardiogram data and photoplethysmogram data to a processing device for analysis and monitoring of health-related metrics.
